# EUROPEAN PATENT APPLICATION

(11) **EP 2 480 020 A1**
(43) Date of publication of application: **25.07.2012**
(21) Application number: 11010025.2
(22) Date of filing: 20.12.2011
(51) Int. Cl.: H04W 12/12, H04L 29/06

(54) **Notification generation by and control of a portable device based on the gait of a person**

(30) Priority: 25.01.2011 US 201113012914
(71) Applicant: Harris Corporation, Melbourne FL 32919 (US)
(72) Inventor: Jastram, Robert, Asheville North Carolina 28803 (US)
(74) Representative: Schmidt, Steffen

(57) **Abstract**

Method and system for gait controlled operations of a device including one or more gait sensors (22) for sensing a pattern of self-propelled movement of a person, which defines a gait. The gait controlled device (10) includes at least one data processor (40) configured for determining one or more first parameters which define a first gait of an authenticated person based on a first output of the one or more gait sensors. The data processor uses a second output of the one or more gait sensors (22) to determine one or more second parameters which define a second gait of a second person. Thereafter, the data processor compares the first parameters to the second parameters to determine if the second gait resulted from the authenticated person. The data processor is configured to perform at least one action responsive to the comparing.

## Description

### Statement of the Technical Field

The inventive arrangements relate to methods and systems for triggering an alert, and more particularly to alert triggering based on gait information.

### Description of the Related Art

So-called "man-down" devices are known in the art for use in generating automatic emergency notifications and alerts. In basic systems, a tilt-switch and a timer are sometimes used to detect when an orientation of a device is in an unexpected condition for a prolonged period of time. For example, if a portable radio device is normally carried in a vertical orientation, then prolonged orientation of the radio in a horizontal position can sometimes suggest that a user has fallen or has been incapacitated. Others have proposed more sophisticated approaches in which a fall is detected by means of a distinctive "motion signature" associated with falling. For example, such a system is disclosed in U.S. Patent No. 6,160,478 to Jacobsen, et al. In Jacobsen et al., one or more accelerometers or impact detectors is used to generate sensor information, which is then evaluated to detect a motion signature associated with a fall. Jacobsen notes that the system can also be used to determine whether a monitored individual is walking, running or staying relatively still.

Still, there is a continuing need to derive useful information from portable equipment. Such information can have implications with regard to both safety and security of users. Such information can also be useful for command and control administrators for personnel management and tactical considerations.

### SUMMARY OF THE INVENTION

The invention concerns a method and system for a gait controlled device. The method includes determining a first pattern of self-propelled movement (e.g., walking, jogging or running) defining a first gait of an authenticated person. The first gait is compared to a sensed pattern of self-propelled movement of a second person, which defines a second gait, to determine if the second person is the authenticated person. Thereafter, the method includes performing at least one action with the device responsive to the comparing step. For example, the action can include disabling one or more device functions, enabling one or more of the device functions, and generating a notification.

In some embodiments, the device includes a memory containing a first pattern of self-propelled movement defining a first gait of an authenticated person. One or more gait sensors (e.g., an accelerometer and/or gyroscope) are provided for sensing a pattern of self-propelled movement of a second person, which defines a second gait. At least one data processor is provided for comparing the first gait to the second gait, to determine if the second person is the authenticated person, and performing at least one action responsive to the comparing. For example, the at least one action can include disabling one or more functions of the gait controlled device, enabling one or more of the functions, and generating a notification using the gait controlled device.

The device can include a portable radio transceiver, in which case the one or more device functions that are disabled can be selected from the group consisting of a receive capability, a transmit capability, an encryption capability, and a GPS reporting capability. According to another aspect of the invention, one or more functional capabilities of the gait controlled device can be temporarily disabled by the data processor until a user input is provided which identifies a user as the authenticated person. The user input can be chosen to include a biometric information and/or a password. Notably, the data processor can also be configured to use the one or more gait sensors of the gait controlled device to learn the first gait. In some embodiments, the data processor is further configured to determine a health status of the authenticated person based on the comparing.

According to another aspect, the invention can include a gait controlled device including one or more gait sensors for sensing a pattern of self-propelled movement of a person, which defines a gait. The gait controlled device includes at least one data processor configured for determining one or more first parameters which define a first gait of an authenticated person based on a first output of the one or more gait sensors. The data processor stores the one or more first parameters in a memory location. The data processor is further configured to use a second output of the one or more gait sensors to determine one or more second parameters which define a second gait of a second person. The data processor is also configured to compare the first parameters to the second parameters to determine if the second gait resulted from the authenticated person. The data processor is configured to perform at least one action responsive to the comparing. For example, the at least one action can include disabling one or more functions of the gait controlled device, enabling one or more of the functions, and generating a notification using the gait controlled device.

### BRIEF DESCRIPTION OF THE DRAWINGS

Embodiments will be described with reference to the following drawing figures, in which like numerals represent like items throughout the figures, and in which:

FIG. 1 is a perspective view of a gait controlled portable electronic device that is useful for understanding the present invention.

FIG. 2 is a block diagram that is useful for understanding the gait controlled portable electronic device in FIG. 1.

FIG. 3 is a flowchart that is useful for understanding a method for using gait to control a portable electronic device.

### DETAILED DESCRIPTION

The present invention is described with reference to the attached figures, wherein like reference numbers are used throughout the figures to designate similar or equivalent elements. The figures are not drawn to scale and they are provided merely to illustrate the present invention. Several aspects of the invention are described below with reference to example applications for illustration. It should be understood that numerous specific details, relationships, and methods are set forth to provide a full understanding of the invention. One having ordinary skill(s) in the relevant art, however, will readily recognize that the invention can be practiced without one or more of the specific details or with other methods. In other instances, well-known structures or operation are not shown in detail to avoid obscuring the invention. The present invention is not limited by the illustrated ordering of acts or events, as some acts may occur in different orders and/or concurrently with other acts or events. Furthermore, not all illustrated acts or events are required to implement a methodology in accordance with the present invention.

The invention concerns a system and method for controlling a device. A pattern of self-propelled movement defines a gait of an authenticated person. The pattern is learned and stored in a memory location. The self propelled movement can include walking, jogging, and/or running. The pattern can include any combination of cyclical forces, accelerations and/or vibrations resulting over a period of time from limb movement, torso movement, head movement, and/or contact with a surface (e.g., the ground or a floor) which result from such self propelled movement. The pattern is detected by one or more sensors attached to the person, and then stored in a memory location as an authentic gait for that person.

Thereafter, a device can be worn which is capable of sensing the pattern associated with the gait of the person. In some embodiments, this can be the same device that is used to learn the pattern defining the gait of the authenticated person; however the invention is not limited in this regard and alternative devices can be used to learn the pattern. The device for sensing the pattern can include one or more sensors which can be used to sense a second gait defined by similar patterns resulting from self-propelled movement of a person. The device can include one or more data stores and suitable processing facilities which allow the authentic gait to be compared to the second gait. The comparing process allows the device to determine if the authentic gait and the second gait are the same. If so, then it can be assumed that the device is being worn by the authenticated person. However, if the second gait is not the same as the authentic gait, then the device performs at least one action.

Generally action performed by the device will involve disabling one or more of the device functions, enabling one or more of the device functions, and/or generating a notification. In general, if a measured gait is not consistent with the authentic gait, an assumption can be made that the device is being worn by an unauthorized person. Accordingly, actions performed by the device can include various steps which prevent unauthorized use of the device, prevent information from being extracted from the device, or facilitate locating of the device.

For example, if the device includes a portable radio transceiver, then various functions which can be disabled include a receive capability, a transmit capability, an encryption capability, and/or a GPS reporting capability. If the device is a computer processing device which contains information, access to such information can be disabled. Alternatively, there may be scenarios where it is desirable to turn on certain function when a gait is not authenticated. For example, it some scenarios it can be advantageous to turn on a transmitter so that a central station can hear what is occurring at the location of the device. In still other embodiments, it can be advantageous to turn on a GPS reporting capability of the device so that its location can be tracked. It can also be desirable to send a notification message to a central station when a measured gait does not match a gait of an authenticated person. The central station can thereafter communicate with the person in possession of the device to determine if they are the authentic person. If the device is a weapon, the weapon can be automatically disabled.

In some instances, an erroneous determination can be made when comparing the sensed or measured gait to the authenticated gait. Consequently, the device can be disabled although it is still in possession of the authenticated person. In such cases, the device can provide a method or mechanism to allow the user to authenticate themselves by other means. For example, the user can enter a password using a user interface provided on the device. Alternatively, the user can provide one or more items of biometric information to authenticate their identity. For example, a fingerprint scan or retinal scan can be performed for this purpose. Once the user has authenticated themselves by other means, the various functions of the device can be restored to their normal operation.

If a determination is made that the gait of the person wearing the device is sufficiently similar to the gait of the authenticated person, then other steps can be performed. For example, the measured gait can be further analyzed to detect additional information. For example, such additional information could include determining the occurrence of a fall resulting from the individual or radio landing abruptly, or a determination that the individual is running - which suggests a potential emergency situation. Gait analysis could also be used to detect an injury in those instances where the measured gait is determined to be authentic, but still at some degree of statistical variance with respect to the authentic gait. Any of these conditions can be used to trigger a suitable notification message transmitted from the device to a central station.

The authenticated gait information can be stored at a memory location in the device or it can be stored remotely. The measured or sensed gait information can be compared with the authenticated gait information using on-board processing facilities included in the device. Alternatively, the sensed or measured gait information can be communicated to the remote location where the such information can be compared with the authenticated gait information.

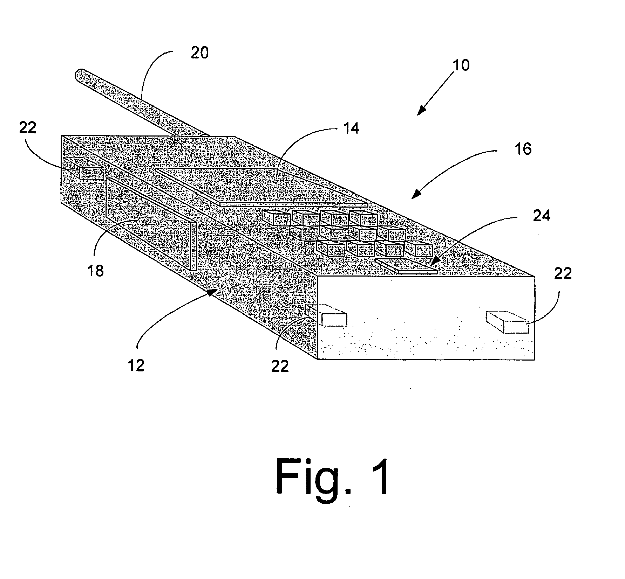

Referring now to FIG. 1, there is shown a portable electronic device 10 which can be worn or carried by a person. The portable device 20 is comprised of a body 12 in which one or more gait sensors 22 can be provided for sensing or measuring a gait of a person on which the device is carried or worn. The portable electronic device can include one or more user interface elements including a keyboard 16, a push-to-talk switch 18, an alpha-numeric display screen 14. If the portable electronic device 10 is a radio transceiver, then an antenna 20 can be provided to facilitate wireless communications. A biometric sensor 24 can also be provided.

The one or more gait sensors 22 can include any sensing or measuring device capable of detecting a pattern of self-propelled movement which defines a gait of a person. The pattern can include any combination of forces, accelerations and/or vibrations resulting over a period of time from limb movement, torso movement, head movement, and/or contact with a surface (e.g., the ground or a floor) which result from such self propelled movement. Accordingly, the gait sensors 22 can include one or more force sensors, accelerometers, gyroscopes, vibration sensors and/or tilt sensors. According to some embodiments, the gait sensor can include a multidimensional accelerometer capable of measuring acceleration in more than one direction. Alternatively, the gait sensor 22 could be comprised of a plurality of accelerometers positioned in different orientations such that an acceleration in any direction can be extrapolated by the accelerations indicated by each of the accelerometers.

The gait sensors can also include an impact detector, which is an accelerometer, gyroscope or a body-linked microphone that detects and interprets the sounds, or rapid accelerations and decelerations that occur on parts of the body remote from the site of impact when an impact occurs, or the sound produced by the shock waves from impact. One such example is the device as developed by Natick Labs in the Future Force Warrior Project that detects shock waves from bullets impacting on the human body and that carry through the bones and viscera. The purpose of the impact sensor will be more apparent as the discussion progresses. In some embodiments, information provided by gait sensors 22 can be supplemented with additional information provided by a GPS device. The GPS device can be useful for measuring a velocity associated with a sensed gait.

It should be understood that one or more different types of sensor can be used concurrently in combination. In other words, gait sensors 22 can include one or more of the different types of sensors described herein, or several of the same type of sensors, some of which may be oriented in different locations or different positions on the electronic device.

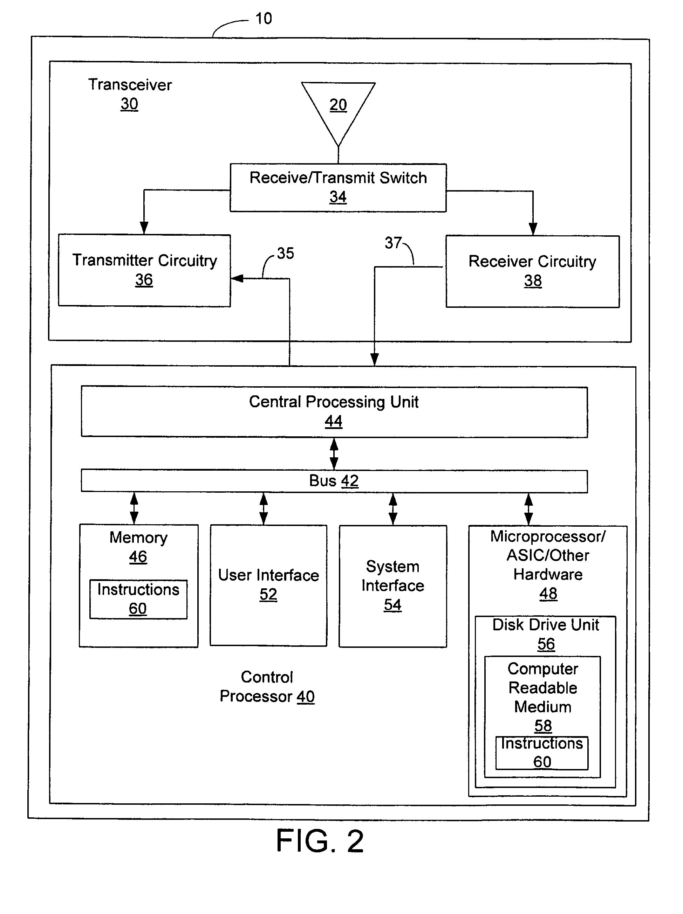

Referring now to FIG. 2, the portable electronic device 10 will be described in further detail. The portable electronic device 30 can include more or less components than those shown in FIG. 2. However, the components shown are sufficient to disclose an illustrative embodiment implementing the present invention.

As shown in FIG. 2, the control processor 40 comprises a system interface 54, a user interface 52, a Central Processing Unit (CPU) 44, a system bus 42, a memory 46 connected to and accessible by other portions of the control processor 40 through system bus 42, and hardware entities 48 connected to system bus 42. System interface 54 allows the control processor 40 to communicate directly with gait sensors 22, biometric sensor 24, and any onboard GPS devices (not shown). At least some of the hardware entities 48 perform actions involving access to and use of memory 46, which may be a random access memory (RAM), a disk drive, and/or a compact disc read only memory (CD-ROM).

Hardware entities 48 may include microprocessors, application specific integrated circuits (ASICs) and other hardware. Hardware entities 48 may include a microprocessor programmed for facilitating the provision of data communication services and processing. The communication services can include, but are not limited to, signal receiving operations, signal processing operations, signal generation operations, and signal communication operations. Gait sensing, processing and analysis methods described herein can be performed by CPU 44, hardware entities 48, or a combination thereof.

As shown in FIG. 2, the hardware entities 48 can include a disk drive unit 56 comprising a computer-readable storage medium 58 on which is stored one or more sets of instructions 60 (e.g., software code) which can be configured to implement one or more of the methodologies, procedures, or functions described herein. The instructions 60 can also reside, completely or at least partially, within the memory 46 and/or within the CPU 44 during execution thereof. The memory 46 and the CPU 44 also can constitute machine-readable media. The term "machine-readable media", as used herein, refers to a single medium or multiple media (e.g., a centralized or distributed database, and/or associated caches and servers) that store the one or more sets of instructions 60. The term "machine-readable media", as used here, also refers to any medium that is capable of storing, encoding or carrying a set of instructions 60 for execution that cause the control processor 40 to perform any one or more of the methodologies of the present disclosure.

The transceiver 30 comprises an antenna 20 for receiving and transmitting Radio Frequency (RF) signals. A receive/transmit (Rx/Tx) switch 34 selectively couples the antenna 20 to the transmitter circuitry 36 and receiver circuitry 38 in a manner familiar to those skilled in the art. The receiver circuitry 38 decodes the RF signals received from a communication device (e.g., a central station transceiver) to derive information therefrom. The receiver circuitry 38 is coupled to control processor 40 via an electrical connection 37. The receiver circuitry 38 provides decoded RF signal information to the control processor 40. The control processor 40 uses the decoded RF signal information in accordance with the function(s) of the invention as described herein.

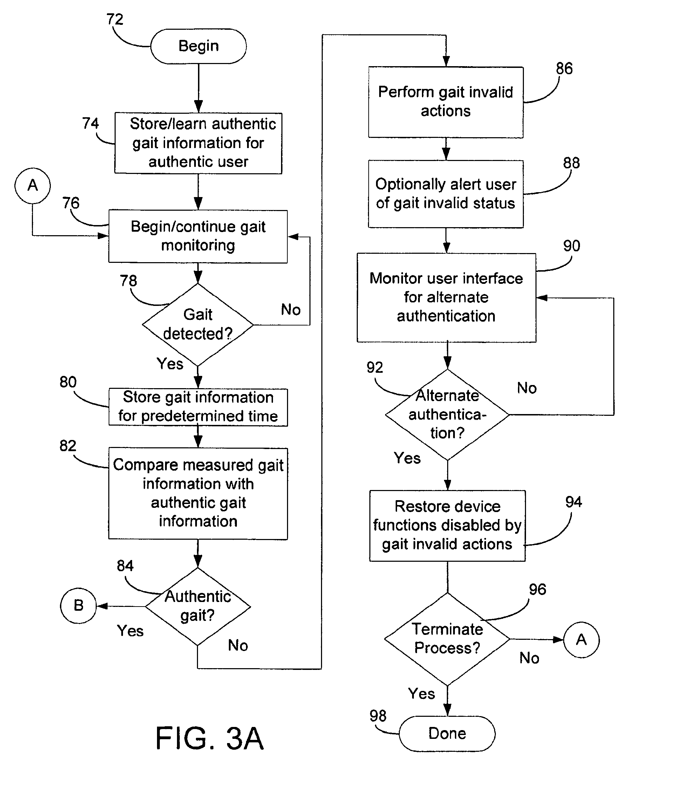

As evident from the above discussion, the control processor 40 implements one or more method embodiments of the present invention. Referring now to FIG. 3A, the processing and control methods of the present invention will now be described in further detail. The process begins with step 72 and continues with step 74. In step 74, the authentic gait information for an user is learned and then stored. The learning process can be performed with device 10, or a dedicated device can be used for this purpose. In either case, the data concerning the authentic gait is stored in a memory location. For example, the authentic gait information can be stored in memory 46 or computer readable medium 58. The gait information can be stored in any suitable format. In some embodiments, raw or unprocessed gait information can be stored; however it can be advantageous to preprocess the gait information to derive one or more statistical or analytical parameters that can be useful for comparing the authentic gait to subsequently measured or sensed gaits as hereinafter described.

In step 76, the gait monitoring process begins. This monitoring process can include buffering sensor data in a memory and performing a preliminary analysis to determine if gait information is available. In some instances, the device 10 may be in a stationary position and the preliminary analysis can determine that gait information is not available, thereby avoiding unnecessary processing. If no gait is detected (78: No) then the process returns to step 76 and continues monitoring. However, if in step 78 a determination is made that gait sensors 22 are sensing movement or acceleration which could be potentially be indicative of a measured or sensed gait (78: Yes), then the process continues on to step 80.

In step 80, gait information is stored in a memory location (e.g., 46, 48). Thereafter, the gait information is processed and analyzed in step 82 to compare the measured or sensed gait information to the authentic gait information. In some embodiments, an authentic gait determination (84: Yes) can be output even if the measured gait data is not statistically identical to the authentic gait information. This can ensure that minor statistical variations in a user's gait do not result in false indications of an invalid gait (84: No). Suitable thresholds can be chosen for one or more statistical parameters to determine whether an authentic gait has been detected. For example, these thresholds can be determined empirically or by using computer simulation and analysis. If as a result of such comparison in step 82, it is determined that the sensed or measured gait data is sufficiently statistically similar to the authentic gait data (84: Yes), then the process continues to step 102. However, if it is determined that the measured or sensed gait information is not sufficiently similar to the authentic gait (84: No), then the process continues on to step 86.

In step 86 gait invalid actions are performed. As discussed above, gait invalid actions can include any disabling one or more functions of device 10, or enabling one or more of functions of device 10, and/or generating a notification using device 10 and transceiver 30.

In step 88 the electronic device 10 can optionally generate an alert 88 to inform a person of the gait invalid status as an indication that the electronic device 10 has detected that the sensed gait and the authentic gait are not sufficiently the same. This alert can be an audio alert generated by device 10, a vibrator type alert, or an alpha-numeric alert communicated on display screen 14, or both. The alert can serve as an indication that alternate authentication is necessary in order to restore functions that were disabled in step 86. Thereafter, in step 90, the electronic device can monitor the device user interface (e.g., keyboard 16 or biometric sensor 24) to determine if alternate authentication information has been provided. The alternate authentication information can be any suitable information that is unique to the authentic user and/or uniquely known to the user and the electronic device. For example, a password and/or biometric information (fingerprint, retinal scan, voice print) can be used for this purpose. If no suitable alternate authentication is received, then the electronic device 10 returns to 90 and continues monitoring the user interface. However, if a suitable alternate authentication is received in step 92 (92: Yes), then the process continues on to step 94. In step 94, device functions disabled by the gait invalid actions are restored or enabled. Thereafter, the process continues on to step 96 and a determination is made as to whether the process should continue or terminate. If the process continues (96: No), it returns to step 76. Otherwise, the process will terminate at 98.

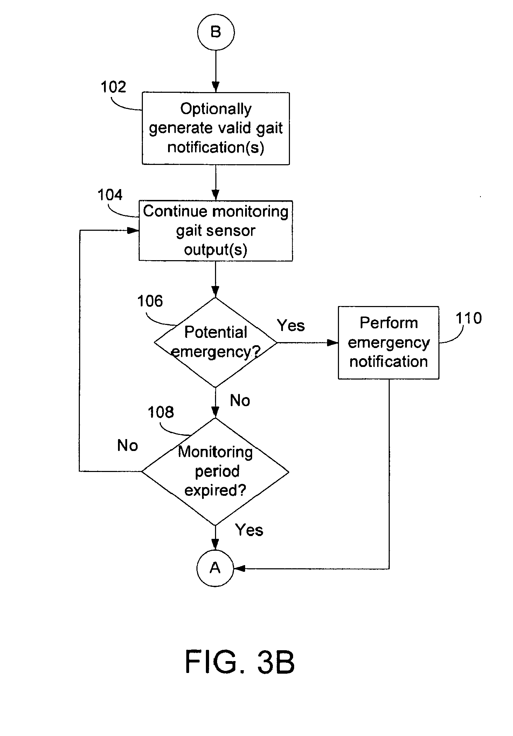

Referring once again to decision block 84, if a sensed or measured gait is detected (84: Yes) then the process continues to optional step 102 in FIG. 3(B) at which a valid gait notification is generated. Such valid gait notification message can be communicated (e.g., by a wireless communication) to a central station as confirmation that the previously authenticated user is in possession of the electronic device and is moving. Recall that the comparing in step 82 can include a comparison of the measured or sensed gait to a walking gait, a jogging gait, and a running gait. Accordingly, the valid gait notification at 102 can also specify whether the movement of the authentic person is a walking movement, a running movement, or a jogging movement, depending on the result of the gait analysis performed in step 82.

The comparing step can also result in a determination that a gait is sufficiently similar to an authentic gait, but that there is some anomaly in the measured or sensed gait information suggests the user is having some difficulty moving. For example, the walking pattern could be substantially identical to the authentic gait, but substantially slower than usual. Alternatively, the gait could be generally consistent with the authentic gait, except for a small variation in movement during one part of the walking, running or jogging process. This could be understood as indicating that the user is authentic, but that some injury or health condition has arisen that has caused the minor gait variation. Accordingly, the valid gait notification message in step 102 could include information indicating a health status of the user, e.g., that the user may be injured or fatigued.

Thereafter, the electronic device can perform further evaluation of the gait sensor(s) output in steps 104, 106. For example, information from the one or more gait sensors 22 can be analyzed to determine a potential emergency condition. The invention is not limited with regard to the types of emergency conditions that can be identified. Recall that the gait sensors 22 of the present invention can include any one of several different kinds of sensors. Accordingly, various different types of emergency conditions can potentially be reported. For example, in some embodiments the electronic device can be programmed to identify an emergency condition when the electronic device recognizes a distinctive "motion signature" associated with a particular event. Such motion signature can be identified by any suitable processing, such as by applying a statistical analysis to the gait sensor output data. Techniques for performing such analysis are described in detail in U.S. Patent Publication No. 2006-0282021 to DeVaul, et al; and in U.S. Patent No. 2008-0129518 to Carlton-Foss. The disclosure of each of these references is incorporated herein by reference. Impact sensors can be used to determine that a person wearing the electronic device has been struck by an object, such as a bullet. Still, the invention is not limited in this regard.

If an analysis of the gait sensor output suggest that an emergency or potential emergency situation exists (106: Yes), then an emergency notification message can be generated at 110. If no emergency situation is detected, then a determination can be made in step 108 as to whether a predetermined monitoring period has expired. The monitoring period can be any suitable length of time. If the monitoring period has not expired, then the process can return to step 104 for further monitoring of gait sensor outputs. However, if the monitoring period has expired, then the method can return to step 76 where a user's gait is once again analyzed and then authenticated.

## Claims

1. A method for controlling a device, comprising:
determining a first pattern of self-propelled movement defining a first gait of an authenticated person;
comparing said first gait to a sensed pattern of self-propelled movement of a second person, which defines a second gait, to determine if said second person is said authenticated person;
performing at least one action with said device responsive to said comparing step.

2. The method according to claim 1, wherein said at least one action is selected from the group consisting of disabling one or more device functions, enabling one or more of said device functions, and generating a notification.

3. The method according to claim 1, wherein said at least one action comprises temporarily disabling one or more functional capabilities of said device until a user input is provided which identifies a user as said authenticated person.

4. The method according to claim 1, wherein said determining step comprises using one or more sensors of said device to learn said first gait.

5. The method according to claim 1, further comprising storing said first gait at a memory location in said device.

6. A gait controlled device, comprising:
a memory containing a first pattern of self-propelled movement defining a first gait of an authenticated person;
one or more gait sensors for sensing a pattern of self-propelled movement of a second person, which defines a second gait;
at least one data processor configured for comparing said first gait to said second gait, to determine if said second person is said authenticated person, and performing at least one action responsive to said comparing.

7. The gait controlled device according to claim 6, wherein said at least one action is selected from the group consisting of disabling one or more functions of said gait controlled device, enabling one or more of said functions, and generating a notification using said gait controlled device.

8. The gait controlled device according to claim 6, wherein said at least one action comprises temporarily disabling one or more functional capabilities of said gait controlled device until a user input is provided which identifies a user as said authenticated person.

9. The gait controlled device according to claim 6, wherein said at least one data processor is configured to use said one or more gait sensors of said gait controlled device to learn said first gait.

10. The gait controlled device according to claim 6, wherein said first gait includes a plurality of gaits of said authenticated person selected from the group consisting of gaits for walking, jogging, and running.
